# EUROPEAN PATENT APPLICATION

(11) **EP 4 350 700 A1**
(43) Date of publication of application: **10.04.2024**
(21) Application number: 22815593.3
(22) Date of filing: 28.02.2022
(51) Int. Cl.: G16C 20/30, C08K 3/016, C08L 101/00, G01N 33/44

(54) **FLAME RESISTANCE PREDICTING DEVICE, FLAME RESISTANCE PREDICTION MODEL GENERATING DEVICE, FLAME RESISTANCE PREDICTION MODEL, AND FEATURE AMOUNT EXTRACTING DEVICE**

(30) Priority: 02.06.2021 JP 2021093236
(71) Applicant: Konica Minolta, Inc., Tokyo 100-7015 (JP)
(72) Inventor: NAKAMURA Yuta, Tokyo 100-7015 (JP); SAITA Yasuharu, Tokyo 100-7015 (JP); IKEDA Yuko, Tokyo 100-7015 (JP); KOJIMA Takeshi, Tokyo 100-7015 (JP)
(74) Representative: MFG Patentanwälte Meyer-Wildhagen Meggle-Freund Gerhard PartG mbB
(86) International application number: PCT/JP2022/008355
(87) International publication number: WO 2022/254835

(57) **Abstract**

A flame resistance predicting device (100) includes: an information acquisition unit (111) that receives an input of material formulation information regarding a material of a polymer composite material; and a prediction unit (113) that predicts information regarding flame resistance of the polymer composite material from the input material formulation information of the polymer composite material using a flame resistance prediction model (prediction model (121)) that predicts the information regarding the flame resistance of the polymer composite material. Examples of the material formulation information include information regarding the kind of resin, the kind of additive, a ratio between the resin and the additive, a structure of the resin, a structure of the additive, and a molding process of the polymer composite material.

## Description

### Technical Field

The present invention relates to a flame resistance predicting device that predicts flame resistance of a polymer composite material from formulation information of a material, a flame resistance prediction model generating device, a flame resistance prediction model, and a feature amount extracting device.

### Background Art

With development of society and advancement of technology, metal is being replaced with plastic in applications such as an automobile, an electronic device, and an industrial material. There is an increasing need to impart flame resistance to plastics more than ever before from a viewpoint of recent sustainable development goals (SDGs) and safety and security. In particular, electric and electronic devices potentially have a risk of ignition due to a short circuit of a circuit, deterioration, and the like, and thus, it is predicted that a demand for flame resistance of a polymer composite material (polymer organic material) used for a housing of a device and the like will become more severe.

As a combustion mechanism of a polymer composite material, it is known that combustion continues in a cycle including the following (1) to (6) (source: homepage of Flame Retardant Chemicals Association of Japan).
(1) Combustion of combustible gas: supplementation of combustible gas and oxygen
(2) Generation of radiation heat by combustion: temperature rise on organic material surface
(3) Heat Conduction into organic material: temperature rise of organic material
(4) Thermal decomposition of organic material: generation of combustible gas
(5) Diffusion of combustible gas to material surface: diffusion in organic material
(6) Diffusion of combustible gas into combustion field: diffusion in gas phase

Therefore, in order to stop combustion, it is only required to prevent continuation of the above cycle, and imparting of flame resistance is only required to act on any one or more of (1) to (6). For example, a flame retardant based on a combustion mechanism has been designed, such as a halogen compound having an effect of stabilizing active OH radicals by a radical trap effect and blocking oxygen, a phosphorus-based compound that promotes formation of char and a heat insulating layer, a metal hydroxide having an endothermic effect by a dehydration reaction, or a drip prevention agent that suppresses dropping (drip) of a resin material during combustion. Imparting of flame resistance to a composite material is designed by adding these flame retardants.

Meanwhile, since a composite material used for a housing or the like is required to have other characteristics such as impact resistance and moldability in addition to flame resistance, a plurality of additives is often used in combination. However, these characteristics are likely to be trade-off. For example, it is known that toughness and rigidity of a resin are reduced by adding a flame retardant, and Patent Literature 1 discloses a thermoplastic resin composition that solves this problem.

### Citation List

### Patent Literature

Patent Literature 1: JP 2011-144222 A

### Summary of Invention

### Technical Problem

The thermoplastic resin composition described in Patent Literature 1 has excellent flame resistance, can prevent adhesion of a flame retardant to a die during injection molding, and has rigidity, toughness, and heat resistance, but other characteristics are not mentioned. When optimal formulation design of a composite material satisfying various characteristics is performed, trial and error is performed in which an experimental formulation in which a plurality of additives is exquisitely blended is determined on the basis of previous intuition and experience, kneading and molding are actually performed, flame resistance and other evaluation tests are performed, and the formulation is reformulated on the basis of results thereof. However, with such a method, development efficiency is poor.

As described above, it is not easy to develop a composite material having flame resistance. In a case of optimization of one characteristic of flame resistance, material design based on the above-described cycle of (1) to (6) is possible. However, as described above, various additives are required in order to satisfy various characteristics in addition to flame resistance. In composite materials in which kinds, blending, and molding processes of resins and additives to be used are different from each other, combustion behaviors thereof are different from each other, and it is difficult for a human to interpret what has been effective to cause flame resistance. As a result, in development of a composite material imparting flame resistance, formulation is currently determined by trial and error by repeating trial production.

The present invention has been made in view of such a background, and an object of the present invention is to provide a flame resistance predicting device capable of improving efficiency of development of a composite material having flame resistance, a flame resistance prediction model generating device, a flame resistance prediction model, and a feature amount extracting device.

### Solution to Problem

The above object of the present invention is achieved by the following means.
(1) A flame resistance predicting device including: an information acquisition unit that receives an input of material formulation information regarding a material of a polymer composite material; and a prediction unit that predicts information regarding flame resistance of the polymer composite material from the input material formulation information of the polymer composite material using a flame resistance prediction model that predicts information regarding the flame resistance of the polymer composite material.
(2) The flame resistance predicting device according to (1), in which the material formulation information includes at least one piece of information among pieces of information regarding the kind of resin, the kind of additive, a ratio between the resin and the additive, a structure of the resin, a structure of the additive, and a molding process of the polymer composite material.
(3) The flame resistance predicting device according to (2), in which the structure of the resin includes at least one of a chemical structure, a weight average molecular weight, a number average molecular weight, a molecular weight distribution, a degree of copolymerization, and a degree of crosslinking of the resin.
(4) The flame resistance predicting device according to (2), in which the additive contains at least one of a filler, a plasticizer, a colorant, a flame retardant, an ultraviolet absorber, an antioxidant, and an elastomer.
(5) The flame resistance predicting device according to (2), in which the information regarding the molding process includes at least one of a kneading process condition and a molding condition.
(6) The flame resistance predicting device according to (1), in which the information regarding the flame resistance is suitability for a standard regarding the flame resistance.
(7) The flame resistance predicting device according to (1), in which the flame resistance prediction model receives an input of information regarding combustion of the polymer composite material in addition to the material formulation information.
(8) The flame resistance predicting device according to (7), in which the information regarding the combustion is information obtained from a temporal change of the polymer composite material during the combustion.
(9) The flame resistance predicting device according to (7), in which the information regarding the combustion of the polymer composite material to be an input of the flame resistance prediction model is information selected according to an importance with respect to the information regarding the flame resistance to be an output of the flame resistance prediction model.
(10) The flame resistance predicting device according to (1), in which the flame resistance prediction model includes: a first-stage prediction model that predicts information regarding combustion of the polymer composite material from the material formulation information regarding a material of the polymer composite material; and a second-stage prediction model that predicts information regarding flame resistance of the polymer composite material from the information regarding the combustion of the polymer composite material.
(11) A flame resistance prediction model generating device including a learning unit that generates a flame resistance prediction model that predicts information regarding flame resistance of a polymer composite material from input material formulation information regarding a material of the polymer composite material using teacher data in which the material formulation information and the information regarding the flame resistance are associated with each other.
(12) The flame resistance prediction model generating device according to (11), in which the teacher data includes information regarding combustion of the polymer composite material, and the flame resistance prediction model receives an input of the information regarding the combustion of the polymer composite material in addition to the material formulation information.
(13) The flame resistance prediction model generating device according to (12), in which the information regarding the combustion is information obtained from a temporal change of the polymer composite material during the combustion.
(14) The flame resistance prediction model generating device according to (12), further including an importance calculation unit that calculates an importance of the information regarding the combustion with respect to the information regarding the flame resistance to be an output of the flame resistance prediction model, in which the learning unit selects the information regarding the combustion to be an input of the flame resistance prediction model according to the importance to generate the flame resistance prediction model.
(15) A flame resistance prediction model generated by the flame resistance prediction model generating device according to any one of (11) to (14).
(16) A feature amount extracting device including an importance calculation unit that calculates an importance of information regarding combustion of a polymer composite material in a flame resistance prediction model that predicts information regarding flame resistance of the polymer composite material from the information regarding the combustion with respect to the information regarding the flame resistance to be an output of the flame resistance prediction model.

### Advantageous Effects of Invention

According to the present invention, it is possible to provide a flame resistance predicting device capable of improving efficiency of development of a composite material having flame resistance, a flame resistance prediction model generating device, a flame resistance prediction model, and a feature amount extracting device.

### Brief Description of Drawings

Fig. 1 is a functional block diagram of a flame resistance predicting device according to a first embodiment.
Fig. 2 is a data configuration diagram of a material information database according to the first embodiment.
Fig. 3 is a flowchart of a learning process according to the first embodiment.
Fig. 4 is a flowchart of a prediction process according to the first embodiment.
Fig. 5 is a confusion matrix indicating a relationship between actual suitability of a composite material according to the first embodiment for a UL94 V-2 grade and suitability predicted by a prediction model.
Fig. 6 is a confusion matrix indicating a relationship between actual suitability of the composite material according to the first embodiment for a UL94 V-0 grade and suitability predicted by a prediction model.
Fig. 7 is a functional block diagram of a flame resistance predicting device according to a second embodiment.
Fig. 8 is a data configuration diagram of a material information database according to the second embodiment.
Fig. 9 is a diagram illustrating a decision tree.
Fig. 10 is a decision tree for explaining an importance according to the second embodiment.
Fig. 11 is a flowchart of a learning process according to the second embodiment.
Fig. 12 is a confusion matrix indicating a relationship between actual suitability of a composite material according to the second embodiment for a UL94 V-2 grade and suitability predicted by a prediction model.

### Description of Embodiments

Hereinafter, a flame resistance predicting device in a mode (embodiment) for carrying out the present invention will be described. The flame resistance predicting device predicts information regarding flame resistance of a polymer composite material (composite material) from material formulation information (formulation information of a material) of the polymer composite material using a machine learning technique. The material formulation information is a ratio of a resin or an additive contained in the composite material. The information regarding the flame resistance is, for example, suitability for a standard regarding the flame resistance. In order to improve prediction accuracy, information regarding specific combustion may be added to the input of the prediction process in addition to the material formulation information.

By predicting the flame resistance from the material formulation information, a composite material predicted to have flame resistance can be preferentially produced by way of trial and tested for the flame resistance. By performing trial production and test from a candidate with higher possibility, the number of times of trial production and test can be reduced, and development can be made efficient.

### <<First embodiment: configuration of flame resistance predicting device>>

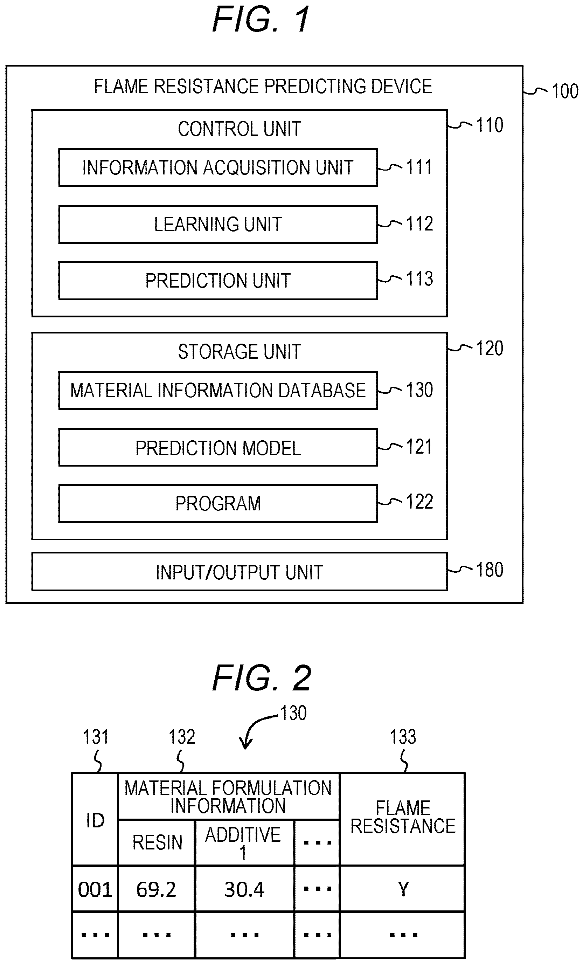

Fig. 1 is a functional block diagram of a flame resistance predicting device 100 according to a first embodiment. The flame resistance predicting device 100 is a computer, and includes a control unit 110, a storage unit 120, and an input/output unit 180. A user interface device such as a display, a keyboard, or a mouse is connected to the input/output unit 180. The input/output unit 180 may include a communication device so as to be able to transmit and receive data to and from another device. In addition, a media drive may be connected to the input/output unit 180 such that data can be exchanged using a recording medium.

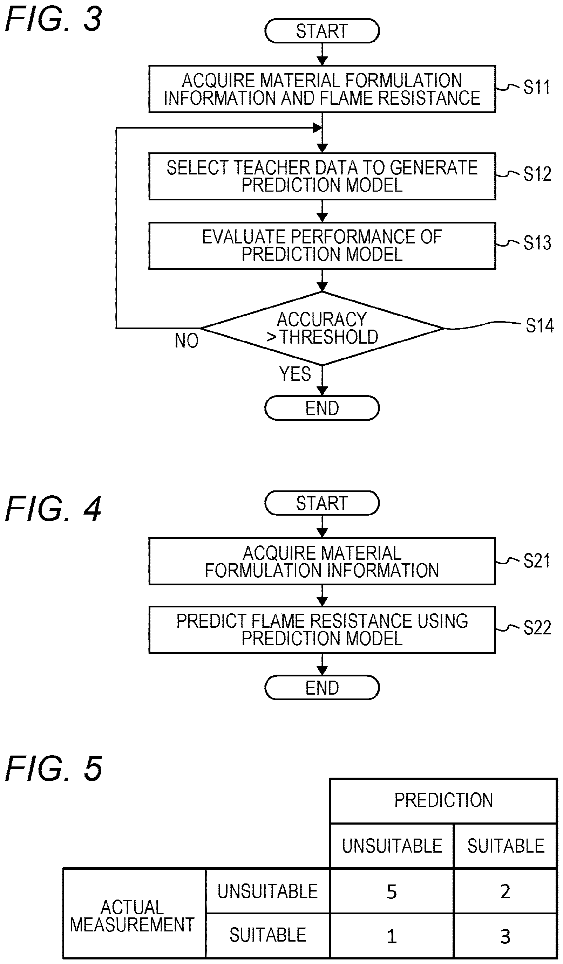

The storage unit 120 includes a storage device such as a read only memory (ROM), a random access memory (RAM), or a solid state drive (SSD). The storage unit 120 stores a material information database 130 (see Fig. 2 described later), a prediction model 121, and a program 122. The prediction model 121 is a machine learning model using material formulation information of a composite material as an explanatory variable, and using suitability (suitable/unsuitable) of the composite material for a standard of flame resistance as an objective variable. The program 122 includes descriptions of a learning process (see Fig. 3 described later) and a prediction process (see Fig. 4 described later) related to the prediction model 121.

### <<First embodiment: material information database>>

Fig. 2 is a data configuration diagram of a material information database 130 according to the first embodiment. The material information database 130 is, for example, tabular data, and one row (record) includes columns (attributes) of identification information 131 (described as ID in Fig. 2), material formulation information 132, and flame resistance 133.

The identification information 131 is identification information of a composite material.

The material formulation information 132 is a ratio of a resin or an additive to be a material of a composite material, and is, for example, a weight ratio. Note that there is generally a plurality of additives.

The flame resistance 133 is suitability of a composite material for a standard of flame resistance, and is "Y" when the composite material is suitable and "N" when the composite material is unsuitable. The flame resistance 133 is information obtained from a result of a combustion experiment performed on a composite material made of a material indicated in the material formulation information 132. The standard of flame resistance is, for example, UL94 related to flame resistance issued by Underwriters Laboratories. The UL94 includes a grade such as V-0 or V-2. The flame resistance 133 indicates suitability of a composite material, for example, for a V-2 grade. The flame resistance 133 may be suitability for another standard, or may be suitability for a unique standard related to flame resistance.

Note that the data (record) in the material information database 130 is used for generation of the prediction model 121 (see step S12 in Fig. 3 described later) and performance evaluation of the prediction model 121 (see step S13). For example, data is appropriately divided into learning data and verification data, a model is generated (trained or learned) with the learning data, and then performance (prediction accuracy) is evaluated with the verification data. The data in the material information database 130 is used, for example, for learning and performance evaluation by a hold-out method or a cross validation method.

### <<First embodiment: control unit>>

Returning to Fig. 1, the control unit 110 includes a central processing unit (CPU), and includes an information acquisition unit 111, a learning unit 112, and a prediction unit 113.

The information acquisition unit 111 acquires material formulation information and flame resistance information of a composite material, and stores the material formulation information and the flame resistance information in the material information database 130.

The learning unit 112 trains a machine learning model using the data in the material information database 130 as teacher data to generate the prediction model 121. The prediction model 121 is, for example, a machine learning model of a decision tree, but may be another model of a machine learning technique, such as a random forest, a neural network, or a support vector machine (SVM).

The prediction unit 113 predicts flame resistance of a composite material from the material formulation information of the composite material acquired by the information acquisition unit 111 using the prediction model 121.

### <<First embodiment: learning process>>

Fig. 3 is a flowchart of a learning process according to the first embodiment.

In step S11, the information acquisition unit 111 acquires material formulation information of a composite material and flame resistance of the composite material, and stores the material formulation information and the flame resistance in the material information database 130. The data of the material information database 130 is data in which the material formulation information 132 is regarded as an explanatory variable (input) and the flame resistance 133 is regarded as an objective variable (output, correct label).

In step S12, the learning unit 112 acquires a plurality of pieces of data in the material information database 130, and trains a machine learning model using these pieces of data as teacher data to generate the prediction model 121. Note that the teacher data is data in which the material formulation information 132 is regarded as an explanatory variable (input) and the flame resistance 133 is regarded as an objective variable (output, correct label).

In step S13, the learning unit 112 evaluates performance (prediction accuracy) of the prediction model 121 using data included in in the material information database 130 and not used in step S12.

In step S14, the learning unit 112 determines whether or not the performance of the prediction model in the step S13 is equal to or more than a predetermined threshold. If the performance is equal to or more than the predetermined threshold (step S14 → YES), the learning unit 112 ends the learning process. If the performance is less than the threshold (step S14 → NO), the learning unit 112 returns to step S12.

### <<First embodiment: prediction process>>

Fig. 4 is a flowchart of a prediction process according to the first embodiment.

In step S21, the information acquisition unit 111 acquires material formulation information of a composite material for which flame resistance is predicted.

In step S22, the prediction unit 113 acquires the material formulation information acquired in step S21 as an input (explanatory variable) of the prediction model 121, and acquires flame resistance to be an output as a prediction result. The prediction unit 113 displays the prediction result on a display connected to the input/output unit 180.

### <<First embodiment: performance evaluation>>

A performance evaluation result of the flame resistance predicting device 100 (prediction model 121) is indicated below. In a first evaluation experiment, 38 composite materials were prepared and subjected to a combustion test to acquire suitability (suitable/unsuitable) thereof for a V2 grade of UL94. Polypropylene was used as a resin, and ten kinds of additives were used. The prediction model 121 of a decision tree was generated using 27 composite materials out of the 38 composite materials as teacher data. Subsequently, performance (prediction accuracy) was evaluated using eleven pieces of verification data that had not been used as teacher data.

Fig. 5 is a confusion matrix indicating a relationship between actual suitability of the composite material according to the first embodiment for a UL94 V-2 grade and suitability predicted by a prediction model. Of the four numerical values, the upper left "5" is the number of composite materials that were predicted to be unsuitable for the V-2 grade, were unsuitable as combustion test results, and were correctly predicted (true negative). The lower right "3" is the number of composite materials that were predicted to be suitable for the V-2 grade, were suitable as combustion test results, and were correctly predicted (true positive).

The top right "2" is the number of composite materials that were predicted to be suitable for the V-2 grade, were unsuitable as combustion test results, and were incorrectly predicted (false positive). The lower left "1" is the number of composite materials that were predicted to be unsuitable for the V-2 grade, were suitable as combustion test results, and were incorrectly predicted (false negative). A correct answer ratio is (5 + 3)/(5 + 3 + 1 + 2) = 72.7%, which is generally good prediction accuracy.

In a second evaluation experiment, 26 composite materials were prepared and subjected to a combustion test to acquire suitability for a V0 grade. Polypropylene was used as a resin, and ten kinds of additives were used. The prediction model 121 of a decision tree was generated using 15 composite materials out of the 26 composite materials as teacher data. Subsequently, performance was evaluated using eleven pieces of verification data that had not been used as teacher data.

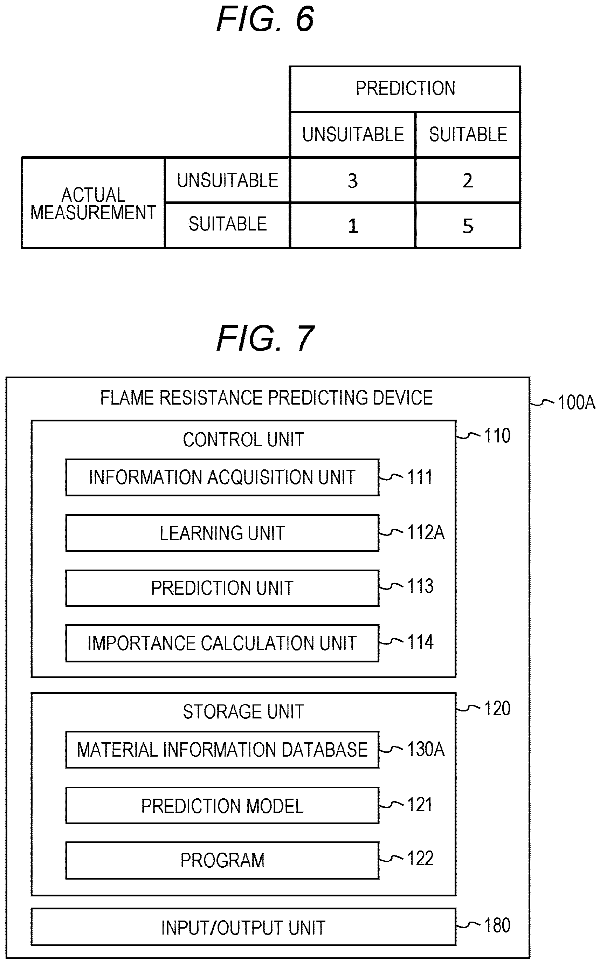

Fig. 6 is a confusion matrix indicating a relationship between actual suitability of the composite material according to the first embodiment for a UL94 V-0 grade and suitability predicted by a prediction model. A correct answer ratio is 72.7%, which is generally good prediction accuracy.

### «Features of first embodiment»

The flame resistance predicting device 100 predicts suitability for a standard of flame resistance from material formulation information of a composite material. By predicting the flame resistance from the material formulation information, a composite material predicted to have flame resistance can be preferentially produced by way of trial and tested for the flame resistance. By performing trial production and test from a candidate with higher possibility, the number of times of trial production and test can be reduced, and development can be made efficient.

### <<Second embodiment: sunimary>>

In the first embodiment, the information (explanatory variable) for predicting the flame resistance is only the material formulation information. Information regarding combustion may be added to the explanatory variable.

Fig. 7 is a functional block diagram of a flame resistance predicting device 100A according to a second embodiment. As compared with the flame resistance predicting device 100 according to the first embodiment, a control unit 110 includes an importance calculation unit 114, and configurations of a learning unit 112A and a material information database 130A are different.

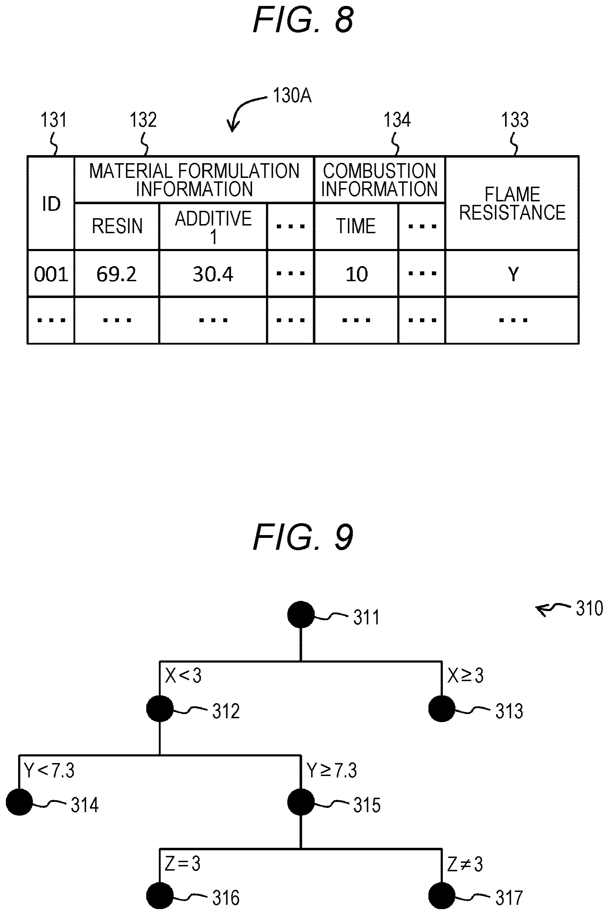

Fig. 8 is a data configuration diagram of the material information database 130A according to the second embodiment. Combustion information 134 that is information regarding combustion of a composite material is added as an attribute. The combustion information 134 is information obtained by preparing and combusting a composite material, and includes information with a lapse of time during combustion (information obtained from a temporal change). Examples of the combustion information include time from flame contact to burning of a composite material, flame spreading manner, flame color, flame size, presence or absence of char (carbonized layer) formation, time until initial drip occurs, the number of times of drip, drip viscosity (high viscosity/low viscosity or the like), color of drip flame, and foaming amount (large/medium/small or the like). Examples of the flame spreading manner include "burnt resin transfers heat" and "flame goes around". The flame size may be expressed by a numerical value of the length thereof or by a ratio thereof to the length of a test piece of a composite material.

Returning to Fig. 7, the description of the difference from the first embodiment will be continued. The importance calculation unit 114 calculates an importance (degree of influence) of each explanatory variable (feature amount) of a prediction model 121 on a prediction result. Hereinafter, a decision tree used to calculate the importance will be described.

Fig. 9 is a diagram illustrating a decision tree 310. Generally, the decision tree is used for regression or classification, but classification (an objective variable is a classification result (category)) will be described below as an example.

Nodes 311 to 317 indicate a group of classification targets. The node 311 which is a route indicates all the classification targets. Classification targets included in the node 311 are classified into a group of classification targets indicated by the node 312 when an explanatory variable X is less than 3, and classified into a group of classification targets indicated by the node 313 when the explanatory variable X is 3 or more. The node 313 is a terminal node in the decision tree 310, and indicates that classification is ended.

Classification targets included in the node 312 are classified into a group of classification targets indicated by the node 314 when an explanatory variable Y is less than 7.3, and classified into a group of classification targets indicated by the node 315 when the explanatory variable Y is 7.3 or more. The node 314 is a terminal node in the decision tree 310, and indicates that classification is ended.

Classification targets included in the node 315 are classified into a group of classification targets indicated by the node 316 when an explanatory variable Z is 3, and classified into a group of classification targets indicated by the node 317 when the explanatory variable Z is not 3. The nodes 316 and 317 are terminal nodes in the decision tree 310, and indicate that classification is ended.

As described above, the group of classification targets indicated by the node 311 is classified into four groups (categories) indicated by the nodes 313, 314, 316, and 317 by the decision tree 310. Note that classification targets included in the nodes 313, 314, 316, and 317 are different from each other, but any nodes may be the same as each other in terms of a classification result (category). For example, it is assumed that the classification target is a composite material, and the classification is determination of suitability for flame resistance. Then, the number of categories is 2, and each of the nodes 313, 314, 316, and 317 is either a suitable node or an unsuitable node.

When the decision tree 310 is constructed, in other words, when an upper node (a group of classification targets) is divided into two lower nodes, an explanatory variable that minimizes a sum of Gini coefficients of the lower nodes and a value thereof are selected as a dividing condition (classification condition). The Gini coefficient indicates a degree of mixing (degree of impurity) of a category (objective variable) of a classification target included in a node. For example, when categories of classification targets included in a certain node are the same, the Gini coefficient is 0. The node in which the Gini coefficient is 0 does not need to be classified and is a terminal of the decision tree 310. In addition, the depth (the number of branches) of the decision tree can be arbitrarily determined, and the Gini coefficient of the terminal node does not necessarily need to be 0. The depth of the decision tree is determined in consideration of, for example, the number of terminal nodes, the number of pieces of data included in each node, and an error ratio.

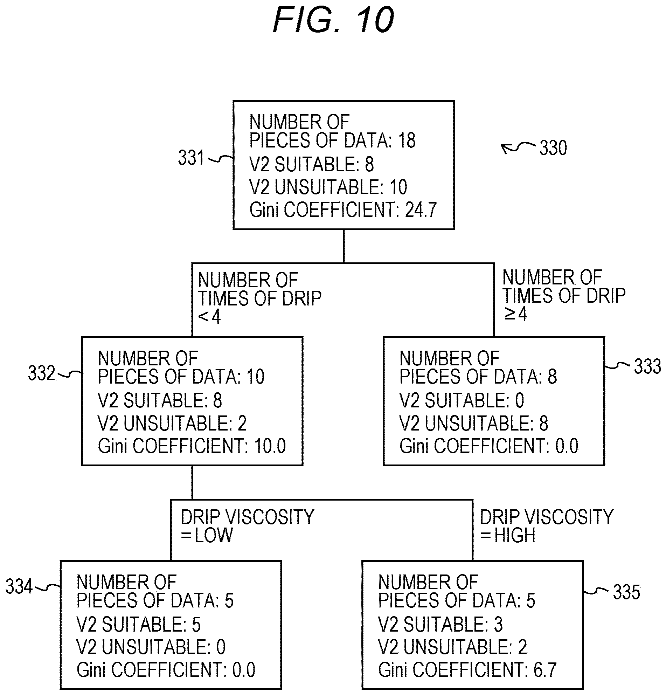

Fig. 10 is a decision tree 330 for explaining an importance according to the second embodiment. The decision tree 330 is the prediction model 121 in a case where the explanatory variable is the combustion information 134 and the flame resistance 133 of the objective variable is suitability for a V-2 grade. In each of nodes 331 to 335, the number of composite materials (the number of pieces of data) included in the node, the number of composite materials suitable for the V-2 grade and the number of composite materials unsuitable for the V-2 grade (V2 suitable/V2 unsuitable), and a Gini coefficient (Gini degree of impurity) are described.

In Fig. 10, the Gini coefficient indicates a degree of mixing (degree of impurity) of suitability of a composite material included in a node for flame resistance. When only suitable composite materials or only unsuitable composite materials are included in a node, the Gini coefficient (degree of impurity) is 0. The lower the Gini coefficient, the higher the degree of only suitable composite materials/only unsuitable composite materials, and a desirable (high correct answer ratio) determination result is obtained. In a process (learning) of constructing the decision tree, a branch condition (an explanatory variable that divides data of an upper node into data of lower nodes and a range of a value thereof) is determined such that a sum of the Gini coefficients of the lower nodes is minimized.

When the decision tree is used as a machine learning model, the importance calculation unit 114 calculates an importance using a Gini coefficient. For example, a difference between the Gini coefficient of an upper node and a sum of Gini coefficients of lower nodes is the importance. The importance calculation unit 114 may calculate the importance with reference to an index used in Boruta, Lasso, or the like in addition to the decision tree. The learning unit 112A generates the prediction model 121 using an explanatory variable with high importance.

Note that the explanatory variable for which the importance calculation unit 114 calculates an importance may be only the combustion information 134 or the material formulation information 132 and the combustion information 134. In addition, when the combustion information 134 includes an explanatory variable with high importance, the learning unit 112A may generate a prediction model using the explanatory variable and the material formulation information 132 as explanatory variables.

### <<Second embodiment: learning process>>

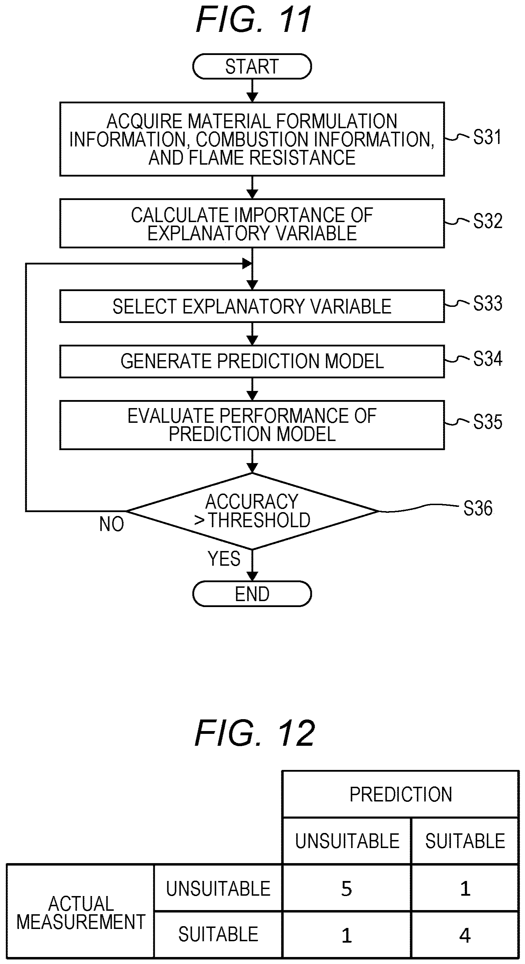

Fig. 11 is a flowchart of a learning process according to the second embodiment. Hereinafter, a process will be described in which the importance calculation unit 114 calculates an importance of an explanatory variable that is the combustion information 134, and the learning unit 112A generates the prediction model 121 using the explanatory variable of the combustion information 134 with high importance and the material formulation information 132 as explanatory variables.

In step S31, the information acquisition unit 111 acquires material formulation information of a composite material, combustion information of the composite material, and flame resistance, and stores the material formulation information, the combustion information, and the flame resistance in the material information database 130A.

In step S32, the importance calculation unit 114 calculates an importance of an explanatory variable (attribute) included in the combustion information 134 (see Fig. 8).

In step S33, the learning unit 112A selects an explanatory variable with high importance. In repetitive processes of steps S33 to S36, for example, the learning unit 112A selects an explanatory variable by sequentially adding one explanatory variable in descending order of importance.

In step S34, the learning unit 112A trains a machine learning model using teacher data using the explanatory variable (attribute) selected in step S33 and the material formulation information 132 as explanatory variables to generate the prediction model 121. The teacher data is acquired from the material information database 130A.

In step S35, the learning unit 112A evaluates performance of the prediction model 121 using data included in in the material information database 130A and not used in step S34.

In step S36, if the performance (prediction accuracy) calculated in step S35 is equal to or less than a predetermined threshold (step S35 → NO), the learning unit 112A returns to step S33, and if the performance exceeds the threshold (step S35 → YES), the learning unit 112A ends the learning process.

### <<Second embodiment: performance evaluation>>

As performance evaluation of the flame resistance predicting device 100A (prediction model 121), an explanatory variable related to combustion with high importance was selected, and then the prediction model 121 using the selected information regarding combustion and the material formulation information 132 as explanatory variables and using the V-2 grade as an objective variable was evaluated.

### <<Second embodiment: performance evaluation: selection of explanatory variable related to combustion with high importance>>

In the selection of an explanatory variable related to combustion, 18 composite materials were prepared and subjected to a combustion test to acquire information regarding various types of combustion and suitability thereof for a V2 grade of UL94. A decision tree using information regarding combustion as an explanatory variable and using flame resistance as an objective variable was created, and an importance was calculated in accordance with Fig. 9. A result thereof is illustrated in Fig. 10.

In order to divide composite materials included in the node 331 by a value of one explanatory variable, attention was paid to the number of times of drip among the explanatory variables. In a case where the composite materials were divided under a condition of whether or not the value is less than 4, a sum of the Gini coefficients of the lower nodes 332 and 333 was minimized. Since the node 333 includes only unsuitable composite materials (Gini coefficient is 0), division is unnecessary.

As for the node 332, in a case where division was performed under a condition of whether a drip viscosity was high or low, a sum of the Gini coefficients of the lower nodes 334 and 335 was minimized. From the above results, in the present example, the number of times of drip and the drip viscosity were selected as explanatory variables with high importance.

### <<Second embodiment: performance evaluation: evaluation result>>

In an evaluation experiment, 38 composite materials were prepared and subjected to a combustion test to acquire the number of times of drip and suitability thereof for a V2 grade of UL94. The material formulation information and the flame resistance are similar to those in the first evaluation experiment in the first embodiment. The prediction model 121 of a decision tree was generated using 27 composite materials out of the 38 composite materials as teacher data. Subsequently, prediction accuracy was evaluated using eleven pieces of verification data that had not been used as teacher data.

Fig. 12 is a confusion matrix indicating a relationship between actual suitability of the composite material according to the second embodiment for a UL94 V-2 grade and suitability predicted by a prediction model. A correct answer ratio is 81.8%, which is better prediction accuracy than that of the first embodiment.

### <<Features of second embodiment>>

The flame resistance predicting device 100A predicts suitability for a standard of flame resistance from material formulation information of a composite material and combustion information. As the combustion information, there are time from flame contact to burning, flame spreading manner, flame color, and the like. By selecting an explanatory variable with high importance, flame resistance can be predicted with high accuracy with a small number of explanatory variables. In the experiment performed this time, it has been confirmed that the importance of the number of times of drip is high and the accuracy is higher than that of the prediction model using only the material formulation information as an explanatory variable.

### <<Modification: prediction from combustion information>>

The explanatory variable of the prediction model in the above-described embodiment includes material formulation information. The flame resistance predicting device may predict the flame resistance using a prediction model that does not include the material formulation information 132 and uses only the combustion information 134 as an explanatory variable.

### <<Modification: Multi-stage prediction model>>

The explanatory variables of the prediction model in the above-described second embodiment are an explanatory variable related to combustion information with high importance and material formulation information. The flame resistance predicting device may predict the combustion information from the material formulation information and predict the flame resistance from the predicted combustion information. Specifically, the flame resistance predicting device may predict the flame resistance from the material formulation information using two prediction models of a first-stage prediction model that predicts the combustion information from the material formulation information and a second-stage prediction model that predicts the flame resistance from the combustion information.

As combustion information that is an objective variable of the first-stage prediction model and is an explanatory variable of the second-stage prediction model, combustion information with high importance (degree of influence) with respect to flame resistance that is a prediction result may be used. Examples of the combustion information with high importance include the number of times of drip and a drip viscosity (see Fig. 10).

The first-stage prediction model can be generated using teacher data using the material formulation information 132 of the material information database 130A (see Fig. 8) as an explanatory variable and using the combustion information 134 as an objective variable. The second-stage prediction model can be generated using teacher data using the combustion information 134 as an explanatory variable and using the flame resistance 133 as an objective variable.

By using such a flame resistance predicting device, the flame resistance can be predicted from the material formulation information without acquiring the number of times of drip and a drip viscosity by preparing a composite material and performing a combustion experiment.

### <<Modification: Material formulation information>>

The material formulation information in the above-described embodiment is a ratio of a resin or an additive to be a material of a composite material, but may include information regarding another material. The material formulation information may include, for example, information regarding the kind of resin, the kind of additive, an addition amount, and a material structure. The material formulation information for the resin may include a weight average molecular weight and a number average molecular weight, a molecular weight distribution, a degree of copolymerization, a degree of crosslinking, a chemical structure, and various other physical property values. The material formulation information for the additive may include various physical property values such as a size and a structure. Note that the additive is a compound other than a polymer resin, the compound affecting characteristics of a polymer composite material to be produced, such as a filler, a plasticizer, a colorant, a flame retardant, an ultraviolet absorber, an antioxidant, or an elastomer. In addition, the material formulation information may include information (molding conditions) regarding a molding process of a composite material, such as a kneading process condition or a molding condition.

### <<Modification: Prediction of other characteristics>>

The above-described flame resistance predicting system predicts information regarding flame resistance, but may predict other characteristics (physical properties) at the same time. In addition to the flame resistance as an objective variable, it is possible to predict other characteristics at the same time as the flame resistance using a prediction model generated from teacher data including characteristics of a composite material, different from the flame resistance. For example, output of toughness and rigidity of a composite resin at the same time as the flame resistance can be utilized for formulation prediction that eliminates trade-off between these characteristics and the flame resistance.

### <<Other Modifications>>

Although some embodiments of the present invention have been described above, these embodiments are merely examples and do not limit the technical scope of the present invention. The flame resistance predicting device 100A according to the second embodiment (see Fig. 7) includes the learning unit 112A, the prediction unit 113, and the importance calculation unit 114, but the learning unit 112A, the prediction unit 113, and the importance calculation unit 114 may be included in different devices, respectively. For example, the flame resistance prediction model generating device may include the learning unit, the feature amount extracting device may include the importance calculation unit, and the flame resistance predicting device may include the prediction unit. In this case, the flame resistance prediction model generating device generates a prediction model with reference to the importance of the explanatory variable calculated by the feature amount extracting device, and the flame resistance predicting device predicts flame resistance using the prediction model.

The present invention can take various other embodiments, and various modifications such as omission and substitution can be made without departing from the gist of the present invention. These embodiments and modifications thereof are included in the scope and gist of the invention described in the present specification and the like, and are included in the invention described in the claims and a scope equivalent thereto.

### <<Resin and additive>>

In the above-described embodiments, polypropylene is mentioned as an example of resin, but other resins and an additive are not mentioned. Hereinafter, a resin and an additive including a filler and a flame retardant will be described.

### <<Resin>>

The resin molded body (composite material) according to the first and second embodiments is a thermoplastic resin, which is a main material constituting the resin molded body. One kind or more kinds of thermoplastic resins may be used. Examples of the thermoplastic resin include a polyolefin, a polycarbonate, polybutylene terephthalate, polyethylene terephthalate, polyphenylene sulfite, polyamide imide, polyether ether ketone, polyether sulfone, a polyimide, a polyvinyl chloride-based resin, a styrene-based resin, a polyamide, a polyacetal-based resin, an acrylic resin, a cellulose-based resin, and a thermoplastic elastomer.

Examples of the polyolefin include polyethylene, an ethylene-vinyl acetate copolymer, a cycloolefin, and polypropylene. Examples of the styrene-based resin include polystyrene, syndiotactic polystyrene, an acrylonitrile-styrene copolymer, and an acrylonitrile-butadiene-styrene copolymer (ABS resin). Examples of the cellulose-based resin include cellulose acetate. Examples of the thermoplastic elastomer include a polystyrene-based thermoplastic elastomer, a polyolefin-based thermoplastic elastomer, a polyurethane-based thermoplastic elastomer, a 1,2-polybutadiene-based thermoplastic elastomer, an ethylene-vinyl acetate copolymer-based thermoplastic elastomer, a fluororubber-based thermoplastic elastomer, and a chlorinated polyethylene-based thermoplastic elastomer.

### «Filler and flame retardant»

The filler can be used without particular limitation, such as a fibrous filler, a needle-shaped filler, or a plate-shaped filler. Examples of the fibrous filler include various inorganic fibers such as a glass fiber, various organic fibers such as an aramid fiber, and a carbon fiber. Examples of the needle-shaped filler include a whisker such as potassium titanate, and a mineral-based needle-shaped filler such as wollastonite. Examples of the plate-shaped filler include mica and talc. Among these, a glass fiber, an aramid fiber, and talc are preferably used from a viewpoint of improving rigidity of a foam molded body.

The flame retardant may be an organic flame retardant or an inorganic flame retardant. Examples of the organic flame retardant include a phosphorus-based compound and a halogen-based compound. Examples of the inorganic flame retardant include an antimony compound and a metal hydroxide.

The phosphorus-based compound easily imparts high flame resistance to a resin composition, and does not have environmental toxicity. The phosphorus-based compound is typically a phosphate compound. Examples of the phosphate compound include a phosphite, a phosphate, and a phosphonate. A phosphate is particularly preferable.

### «Plasticizer, ultraviolet absorber, and antioxidant»

Examples of the plasticizer include an aromatic carboxylate (such as dibutyl phthalate), an aliphatic carboxylate (such as methyl acetyl ricinolate), an aliphatic diarboxylate (such as adipic acid-propylene glycol-based polyester), an aliphatic tricarboxylate (such as triethyl citrate), a phosphoric acid triester (such as triphenyl phosphate), an epoxy fatty acid ester (such as epoxybutyl stearate), and a petroleum resin. Examples of the antioxidant include a hindered phenol-based antioxidant, a sulfur-containing organic compound-based antioxidant, and a phosphorus-containing organic compound-based antioxidant. Examples of the ultraviolet absorber include a benzotriazole-based ultraviolet absorber, a benzophenone-based ultraviolet absorber, and a salicylate-based ultraviolet absorber.

### <<Other components>>

As other components, a reinforcing agent, a dispersant, a pigment or a colorant, a filler, a crystallization accelerator, a transparency agent, an antifoaming agent, a flame retardant aid, an antistatic agent, a processing aid, a lubricant, an organic peroxide, a plasticizer, a photocatalyst, and the like can be used. These other compounds may be used singly or in combination of two or more kinds thereof.

As the reinforcing agent, carbon black, silica, a glass fiber, a carbon fiber, a cellulose fiber, an aramid fiber, or the like can be used.

As the dispersant, a wax, a modified wax, metal soap, a low molecular weight polyethylene, a low molecular weight polypropylene, or the like can be used.

As the wax, a paraffin wax, a polyolefin wax, or the like can be used.

As the modified wax, for example, a wax having an acidic group in a molecular structure can be used, and specific examples thereof include a maleic acid-modified wax, a fumaric acid-modified wax, an acrylic acid-modified wax, a methacrylic acid-modified wax, and a crotonic acid-modified wax.

As the metal soap, a sodium salt, a calcium salt, a zinc salt, a magnesium salt, and an aluminum salt of a fatty acid such as stearic acid, hydroxystearic acid, behenic acid, montanic acid, lauric acid, or sebacic acid can be used.

As the pigment and the colorant, an inorganic pigment and an inorganic pigment can be used. Specific examples of the inorganic pigment include red iron oxide, titanium oxide, cadmium red, cadmium yellow, zinc oxide, ultramarine blue, cobalt blue, calcium carbonate, titanium yellow, white lead, red lead, yellow lead, and Prussian blue. Specific examples of the organic pigment include quinacridone, polyazo yellow, anthraquinone yellow, polyazo red, azo lake yellow, perylene, phthalocyanine blue, phthalocyanine green, and isoindolinone yellow.

### Reference Signs List

100 Flame resistance predicting device (flame resistance prediction model generating device)
100A Flame resistance predicting device (flame resistance prediction model generating device, feature amount extracting device)
111 Information acquisition unit
112, 112ALearning unit
113 Prediction unit
114 Importance calculation unit
121 Prediction model (flame resistance prediction model)
130, 130A Material information database
132 Material formulation information
133 Flame resistance
134 Combustion information

## Claims

1. A flame resistance predicting device comprising:
an information acquisition unit that receives an input of material formulation information regarding a material of a polymer composite material; and
a prediction unit that predicts information regarding flame resistance of the polymer composite material from the input material formulation information of the polymer composite material using a flame resistance prediction model that predicts information regarding the flame resistance of the polymer composite material.

2. The flame resistance predicting device according to claim 1, wherein
the material formulation information includes at least one piece of information among pieces of information regarding the kind of resin, the kind of additive, a ratio between the resin and the additive, a structure of the resin, a structure of the additive, and a molding process of the polymer composite material.

3. The flame resistance predicting device according to claim 2, wherein
the structure of the resin includes at least one of a chemical structure, a weight average molecular weight, a number average molecular weight, a molecular weight distribution, a degree of copolymerization, and a degree of crosslinking of the resin.

4. The flame resistance predicting device according to claim 2, wherein
the additive contains at least one of a filler, a plasticizer, a colorant, a flame retardant, an ultraviolet absorber, an antioxidant, and an elastomer.

5. The flame resistance predicting device according to claim 2, wherein
the information regarding the molding process includes at least one of a kneading process condition and a molding condition.

6. The flame resistance predicting device according to claim 1, wherein
the information regarding the flame resistance is suitability for a standard regarding the flame resistance.

7. The flame resistance predicting device according to claim 1, wherein
the flame resistance prediction model receives an input of information regarding combustion of the polymer composite material in addition to the material formulation information.

8. The flame resistance predicting device according to claim 7, wherein
the information regarding the combustion is information obtained from a temporal change of the polymer composite material during the combustion.

9. The flame resistance predicting device according to claim 7, wherein
the information regarding the combustion of the polymer composite material to be an input of the flame resistance prediction model is information selected according to an importance with respect to the information regarding the flame resistance to be an output of the flame resistance prediction model.

10. The flame resistance predicting device according to claim 1, wherein
the flame resistance prediction model includes:
a first-stage prediction model that predicts information regarding combustion of the polymer composite material from the material formulation information regarding a material of the polymer composite material; and
a second-stage prediction model that predicts information regarding flame resistance of the polymer composite material from the information regarding the combustion of the polymer composite material.

11. A flame resistance prediction model generating device comprising a learning unit that generates a flame resistance prediction model that predicts information regarding flame resistance of a polymer composite material from input material formulation information regarding a material of the polymer composite material using teacher data in which the material formulation information and the information regarding the flame resistance are associated with each other.

12. The flame resistance prediction model generating device according to claim 11, wherein
the teacher data includes information regarding combustion of the polymer composite material, and
the flame resistance prediction model receives an input of the information regarding the combustion of the polymer composite material in addition to the material formulation information.

13. The flame resistance prediction model generating device according to claim 12, wherein
the information regarding the combustion is information obtained from a temporal change of the polymer composite material during the combustion.

14. The flame resistance prediction model generating device according to claim 12, further comprising an importance calculation unit that calculates an importance of the information regarding the combustion with respect to the information regarding the flame resistance to be an output of the flame resistance prediction model, wherein
the learning unit selects the information regarding the combustion to be an input of the flame resistance prediction model according to the importance to generate the flame resistance prediction model.

15. A flame resistance prediction model generated by the flame resistance prediction model generating device according to any one of claims 11 to 14.

16. A feature amount extracting device comprising an importance calculation unit that calculates an importance of information regarding combustion of a polymer composite material in a flame resistance prediction model that predicts information regarding flame resistance of the polymer composite material from the information regarding the combustion with respect to the information regarding the flame resistance to be an output of the flame resistance prediction model.
